# EUROPÄISCHE PATENTANMELDUNG

(11) **EP 0 576 901 A2**
(43) Veröffentlichungstag der Anmeldung: **05.01.1994**
(21) Anmeldenummer: 93109548.3
(22) Anmeldetag: 15.06.1993
(51) Int. Cl.: A61F 13/14, A61F 5/37

(54) **Schultergelenkbandage**

(30) Priorität: 01.07.1992 DE 4221502
(71) Anmelder: Bauerfeind GmbH & Co., D-47880 Kempen (DE)
(72) Erfinder: Bauerfeind, Hans B., D-4152 Kempen 1 (DE); Scheuermann, Rainer, Dr., D-2300 Kiel 14 (DE)
(74) Vertreter: Bardehle, Heinz, Dipl.-Ing.

(57) **Zusammenfassung**

Schultergelenkbandage (2) mit einem den Oberarm (6) umschließenden Schlauchabschnitt (3) und einer sich hieran anschließenden Schulterkappe (4), an deren halsseitigen Rand Haltegurte (8,10) ansetzen, von denen der eine als Rückengurt (10) diagonal den Rücken überquert und unter der Achselhöhle der der Schulterkappe gegenüberliegenden Schulter die Hüfte umfaßt, während der andere mindestens teilweise als Brustgurt (9) über die Brustseite zu dieser Hüfte geführt ist, wo sich Rücken- und Brustgurt (9,10) kreuzen und sich auf der jeweils anderen Körperseite in Richtung auf die andere Hälfte fortsetzen. Beide Fortsetzungen (14,15) umschlingen bei an die betreffende Hüfte angelegtem Oberarm diesen gegensinnig von der Hüftseite her, wobei das Ende (16) jeder Fortsetzung nach Umschlingung des Oberarms jeweils an derselben Fortsetzung befestigt ist.

## Beschreibung

Die Erfindung bezieht sich auf eine Schultergelenkbandage mit einem den Oberarm umschließenden Schlauchabschnitt und einer sich hieran anschließenden Schulterkappe, an deren halsseitigen Rand Haltegurte ansetzen, von denen der eine als Rückengurt diagonal den Rücken überquert und unter der Achselhöhle der der Schulterkappe gegenüberliegenden Schulter die Hüfte umfaßt, während der andere mindestens teilweise als Brustgurt über die Brustseite zu dieser Hüfte geführt ist, wo sich Rücken- und Brustgurt kreuzen und sich auf der jeweils anderen Körper seite in Richtung auf die andere Hälfte fortsetzen.

Eine derartige Schultergelenkbandage ist in dem DE-GBM 90 10 801.9 und der DE-PS 37 04 288 bekannt. Diese Schultergelenkbandage dient dazu, zur Nachbehandlung von operativen Eingriffen am Schultergelenk die verletzte Schulter zu bandagieren, um Schmerzen zu lindern und den Heilungsprozeß zu unterstützen, wobei dem Schultergelenk ein erheblicher Bewegungsspielraum erhalten bleibt.

Der Erfindung liegt die Aufgabe zugrunde, die bekannte Schultergelenkbandage in einer Weise umzugestalten, daß sie im Gegensatz zu der bei der bekannten Schultergelenkbandage verfolgten Zielrichtung eine Ruhigstellung des Schultergelenks bewirkt. Erfindungsgemäß geschieht dies dadurch, daß beide Fortsetzungen bei an die betreffende Hüfte angelegtem Oberarm diesen gegensinnig von der Hüftseite her umschlingen, wobei das Ende jeder Fortsetzung nach Umschlingung des Oberarms jeweils an derselben Fortsetzung befestigt ist.

Die bei der bekannten Schultergelenkbandage lediglich miteinander verbundenen Fortsetzungen werden bei der erfindungsgemäßen Schultergelenkbandage dazu benutzt, durch gegensinnige Umschlingung des Oberarms diesen sowohl an die betreffende Körperseite heranzuziehen als auch in Richtung nach vorne und nach hinten einen gewissen Zug auszuüben, wobei sich diese Kräfte wegen der Umschlingung des Oberarms sowohl von der Brustseite als auch von der Rückenseite her gegenseitig kompensieren, für den Oberarm als Zug insofern nicht in Erscheinung treten. Wegen der gegenseitigen Umschlingung bleibt dabei auch die gegenseitige Fixierung der Fortsetzungen erhalten, obwohl deren Enden jeweils an der selben Fortsetzung befestigt sind, da jede in der Fortsetzung wirkende Zugkraft über den fixierten Oberarm von der jeweils anderen Fortsetzung aufgefangen wird. Um das Anlegen der Schultergelenkbandage zu erleichtern, und eine Anpassung an unterschiedliche Armumfänge zu ermöglichen, gestaltet man diese zweckmäßig so, daß der Schlauchabschnitt, daß der Schlauchabschnitt zur erleichterten Aufnahme des Oberarms längsgeschlitzt ist und entlang des Schlitzes jeweils die andere Seite überdeckende Laschen mit Klettverschluß angeordnet sind.

Um den beiden Haltegurten hinsichtlich ihrer gegenseitigen Lage nach überqueren des Rückens bzw. der Brust zu stabilisieren, kann man die beide Haltegurte an ihrer Kreuzungsstelle in einer Kreuzschnalle gegenseitig halten, und zwar jeweils einzeln gegenüber der Kreuzschnalle verschiebbar. Durch die Verschiebbarkeit jedes einzelnen Haltegurtes läßt sich die Lage der Kreuzungsstelle im Bereich der der Schulterkappe gegenüberliegenden Hüfte in einfacher Weise einstellen, so daß sich für die Länge der Fortsetzungen und deren Umschlingung des Oberarms ausreichende Längen ergeben.

Für die Befestigung der Enden der Fortsetzungen an diesen selbst versieht man die Enden der Fortsetzung zweckmäßig mit einem Klettverschluß.

In diesem Falle gestaltet man die Schultergelenkbandage zweckmäßig aus Flauschmaterial, wobei die Enden der Fortsetzungen mit Klettband versehen sind.

Um auf dem Unterarm zuverlässig zu fixieren, kann man eine Manschette zur Aufnahme des Unterarms vorsehen, die um die bauchseitige Fortsetzung des betreffenden Haltegurts geschlungen ist. Da die bauchseitige Fortsetzung von der Kreuzungsstelle zum umschlungenen Oberarm fest am Bauch anliegt, ist auch die Manschette festgehalten, wodurch ein von der Manschette getragender Unterarm ebenfalls solide fixiert ist.

Um dem Patienten die Möglichkeit zu geben, je nach Art der Behandlung den Unterarm bewegen und wieder fixieren zu können, gestaltet man die Manschette zweckmäßig so, daß diese aus einem Band gebildet ist, dessen Enden mittels eines Klettverschlusses zusammengefügt sind. Bei Öffnen des Klettverschlusses kann die Manschette leicht abgenommen werden und gibt damit dem Unterarm über das Ellenbogengelenk seine Beweglichkeit. Mit Anlegen der Manschette wird dann der Unterarm wieder fixiert.

In den Figuren ist ein Ausführungsbeispiel der Erfindung dargestellt. Es zeigen:
- Figur 1: die Frontansicht eines Patienten mit angelegter Schultergelenkbandage, die einen Schlauchabschnitt und eine anschließende Schulterkappe aufweist,
- Figur 2: eine in der Bandage enthaltene Kreuzschnalle,
- Figur 3: die Rückenansicht eines Patienten,
- Figur 4: die Seitenansicht der Schulterkappe,
- Figur 5: die Frontansicht des Patienten mit der den Unterarm tragenden Manschette.

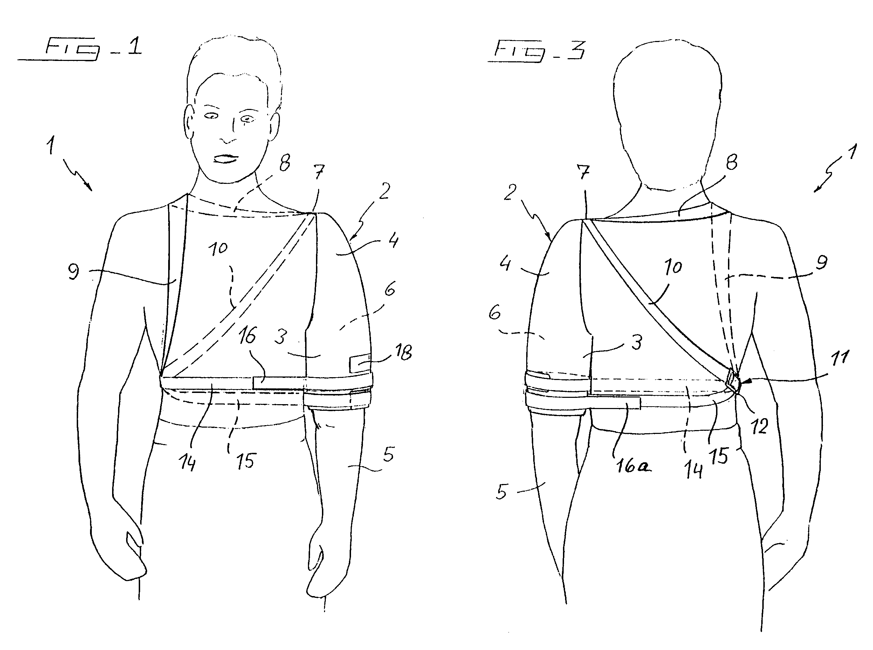

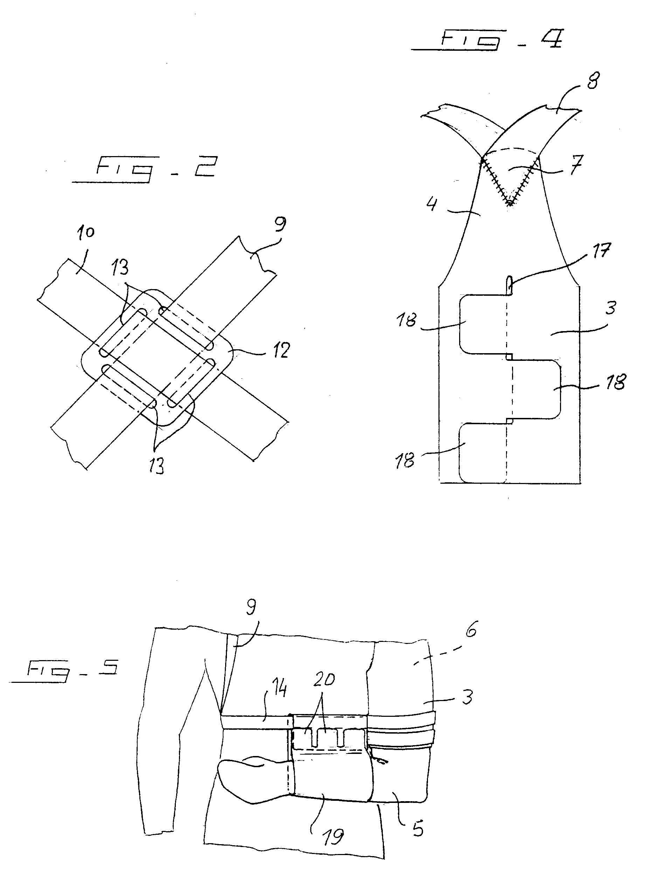

Figur 1 zeigt einen Patienten 1 mit angelegter Schultergelenkbandage 2, die die Schulterkappe 4 enthält. Die Schulterkappe 4 legt sich satt um die Schulter des Patienten 1 und umfaßt mit ihrem Schlauchabschnitt 3 dessen Oberarm. Zum Anziehen der Schultergelenkbandage 2 wird der Unterarm 5 durch den Schlauchabschnitt 3 hindurch geführt, bis dieser in den Bereich des Oberarms 6 gelangt, wobei sich die nach innen hin offene Schulterkappe 4 um die Schulter legt. Um der Schultergelenkbandage 2 eine sicheren Sitz am Körper des Patienten 1 zu geben, sind zwei Haltegurte vorgesehen, die an der Befestigungsstelle 7 zusammen an dem dem Hals zugewandten Rand der Schulterkappe 4 befestigt sind. Von der Befestigungsstelle 7 verläuft der eine Haltegurt 8 unterhalb des Nackens des Patienten 1 zu der der Schulterkappe 4 gegenüberliegenden Schulter des Patienten, wo er in den Brustgurt 9 übergeht. Der andere Haltegurt verläuft von der Befestigungsstelle 7 als diagonal über den Rücken verlaufender Rückengurt 10 zu der dem Schlauchabschnitt 3 gegenüberliegenden Hüfte, wo sich Rückengurt 10 und Brustgurt 9 kreuzen. An dieser Kreuzungsstelle 11 ist eine in der Figur 2 dargestellte Kreuzschnalle 12 vorgesehen, durch die die sich in ihr kreuzenden Gurte also der Rückengurt 9 und der Brustgurt 10, gegenseitig festgehalten werden. Die Kreuzschnalle 12 ist mit vier Langlöchern 13 versehen, wobei jeweils gegenüberliegende Langlöcher zur Einfädelung jeweils eines Gurtes dienen. Hierdurch ist gewährleistet, daß jeder Gurt für sich durch die Kreuzschnalle 12 hindurchgeschoben werden kann, so daß also die Kreuzschnalle 12 gegenüber jedem einzelnen Gurt verschiebbar bleibt.

Von der Kreuzungsstelle 11 setzt sich der Rückengurt 10 in der Fortsetzung 14 fort. Der Brustgurt 9 geht hier in seine Fortsetzung 15 über. Mit diesen beiden Fortsetzungen wird die Ruhigstellung des Oberarms 6 auf folgende Weise herbeigeführt. Die Fortsetzung 14, die über den Bauch des Patienten 1 verläuft, ist von hinten her um den Schlauchabschnitt 3 herumgeschlungen und nach Umschlingung an der Vorderseite der Fortsetzung 14 befestigt. In entsprechender Weise ist die über den Rücken verlaufende Fortsetzung 15 zwischen dem Körper des Patienten 1 und dem Oberarm 6 hindurchgeschlungen und umschlingt daher von vorne her den Schlauchabschnitt 6, wonach das betreffende Ende der Fortsetzung 14 auf der Rückenseite des Patienten an dieser befestigt ist. Auf diese Weise ist der Oberarm 6 des Patienten 1 durch zwei gegensinnig verlaufende Schlaufen gehalten, die einerseits den Oberarm 6 an den Körper des Patienten 1 heranziehen und andererseits verhindern, daß der Patient mit seinem Oberarm 6 etwa eine Schwingbewegung ausführt. Mit den beiden Umschlingungen des Oberarms 6 durch die Verlängerung 14 und 15 wird der Oberarm 6 unter gegenseitger Aufhebung etwaiger Zugkräfte sicher in seiner herabhängenden Lage gehalten.

Figur 3 zeigt die Rückansicht des Patienten 1, aus der die Führung der beiden Haltegurte über den Rücken hervorgeht. Dargestellt ist wieder die Schulterkappe 4 und der Schlauchabschnitt 3 sowie der eine Haltegurt 8 der im Bereich des Nackens des Patienten 1 von der Befestigungsstelle 7 zu der der Schulterkappe 4 gegenüberliegenden Schulter des Patienten verläuft und hier in den gestrichelt gezeichneten Brustgurt 9 übergeht. Von der Befestigungsstelle 7 verläuft diagonal über den Rücken des Patienten 1 der Rückengurt 10, der sich mit dem Brustkorb 9 in der Kreuzungsstelle 11 trifft. Von dieser Kreuzungsstelle aus verläuft die Fortsetzung 14 des Rückengurts 10 über den Bauch des Patienten 1, während die Fortsetzung 15 des Brustgurtes 9 über den Rücken des Patienten 1 verläuft. Im Bereich des Schlauchabschnitts 3 umschlingen dann die Fortsetzungen 14 und 15 den Schlauchabschnitt 3, und zwar derart, daß die Fortsetzung 14 von der Bauchseite her nach hinten hin zwischen dem Schlauchabschnitt und dem Körper des Patienten 1 durchtritt um auf der Rückenseite des Patienten 1 den Schlauchabschnitt 3 zu umschlingen, wonach die Fortsetzung 14 mit ihrem Ende 16 an der Fortsetzung 14 befestigt ist. Umgekehrt tritt die Fortsetzung 15 vom Rücken des Patienten 1 her zwischen dessen Körper und dem Schlauchabschnitt 3 nach vorne durch, um den Schlauchabschnitt 3 zu umschlingen, woraufhin dann die Fortsetzung 15 auf der Frontseite um den Schlauchabschnitt 3 gelegt ist und mit ihrem Ende 16a an der Fortsetzung 15 haftet.

Figur 4 zeigt die Schulterkappe 4 und den Schlauchabschnitt 3 allein Der Schlauchabschnitt 3 ist hier mit dem Längsschlitz 17 versehen, der sich zum leichteren Anlagen der Schultergelenkbandage öffnen läßt. Um den Schlauchabschnitt 3 zu schließen, sind die Laschen 18 vorgesehen, die abwechselnd den Schlitz 17 übergreifen und als Klettverschluß ausgebildet sind, so daß mit Andrücken der Lappen 18 gegen das Material des Schlauchabschnitts 17 dieser geschlossen wird.

In Figur 5 ist die Manschette 19 dargestellt, die zur Ruhigstellung des Unterarms 5 dient und um die bauchseitig verlaufende Fortsetzung 14 geschlungen ist. Die Manschette 19 hängt also gewissermaßen an der Fortsetzung 14. Bei in die Manschette 19 eingesteckem Unterarm 5 ist dieser so festgehalten, daß er praktisch nicht bewegt werden kann. Dies hängt auch von der Straffheit der Umschlingung des Unterarms 5 durch die Manschette 19 ab. Damit die Manschette 19 leicht angelegt werden kann, ist sie nach Art eines Bandes ausgebildet, daß mit seinen Enden durch überlappende Klettverschlüsse zusammengehalten wird. Zum Einschieben des Unterarms 5 werden die Klettverschlüsse geöffnet, woraufhin das betreffende Band als Manschette um den Unterarm geschlungen werden kann, woraufhin mit Schließen der Klettverschlüsse 20 die Manschette sich fest um den Unterarm 5 legt und diesem damit arritiert.

Um die verwendeten Klettverschlüsse und die Befestigung an den Enden der beiden Fortsetzungen 14 und 15 günstig zu gestalten, gestaltet man die Schultergelenkbandage selbst aus Flauschmaterial, das dann mit Kletteilen der Klettverschlüsse 20 bzw. der Lappen 18 zusammenwirkt. Entsprechend sind die Enden der Verlängerungen 14 und 15 mit Klettmaterial zu versehen, das sich dann in dem Flauschmaterial der betreffenden Verlängerung festhakt.

## Patentansprüche

1. Schultergelenkbandage (2) mit einem den Oberarm (6) umschließenden Schlauchabschnitt (3) und einer sich hieran anschließenden Schulterkappe (4), an deren halsseitigen Rand Haltegurte (8,10) ansetzen, von denen der eine als Rückengurt (10) diagonal den Rücken überquert und unter der Achselhöhle der der Schulterkappe (4) gegenüberliegenden Schulter die Hüfte umfaßt, während der andere mindestens teilweise als Brustgurt (9) über die Brustseite zu dieser Hüfte geführt ist, wo sich Rücken- und Brustgurt (9,10) kreuzen und sich auf der jeweils anderen Körperseite in Richtung auf die andere Hälfte fortsetzen, **dadurch gekennzeichnet**, daß beide Fortsetzungen (14,15) bei an die betreffende Hüfte angelegtem Oberarm (6) diesen gegensinnig von der Hüftseite her umschlingen, wobei das Ende (16) jeder Fortsetzung nach Umschlingung des Oberarms (6) jeweils an derselben Fortsetzung (14,15) befestigt ist.

2. Schultergelenkbandage nach Anspruch 1, **dadurch gekennzeichnet**, daß der Schlauchabschnitt (3) zur erleichterten Aufnahme des Oberarms (6) längsgeschlitzt ist und entlang des Schlitzes (17) jeweils die andere Seite überdeckende Laschen (18) mit Klettverschluß angeordnet sind.

3. Schultergelenkbandage nach Anspruch 1 oder 2, **dadurch gekennzeichnet**, daß die beiden Haltegurte (8,10) an ihrer Kreuzungsstelle (11) in einer Kreuzschnalle (12) gegenseitig gehalten sind, und zwar jeweils einzeln gegenüber der Kreuzschnalle (12) verschiebbar.

4. Schultergelenkbandage nach einem der Ansprüche 1 bis 3, **dadurch gekennzeichnet**, daß die Enden (16) der Fortsetzungen (14,15) an diesen mittels Klettverschluß gehalten sind.

5. Schultergelenkbandage nach Anspruch 4, **dadurch gekennzeichnet**, daß sie aus Flauschmaterial besteht und die Enden (16) der Fortsetzungen (14,15) mit Klettband versehen sind.

6. Schultergelenkbandage nach einem der Ansprüche 1 bis 5, **dadurch gekennzeichnet**, daß um die bauchseitige Fortsetzung (14) eine zur Aufnahme des Unterarms (5) dienende Manschette (19) geschlungen ist.

7. Schultergelenkbandage nach Anspruch 6, **dadurch gekennzeichnet**, daß die Manschette (19) aus einem Band gebildet ist, dessen Enden mittels eines Klettverschlusses (20) zusammengefügt sind.
